# EUROPÄISCHE PATENTSCHRIFT

(11) **EP 0 897 302 B1**
(45) Veröffentlichungstag und Bekanntmachung des Hinweises auf die Patenterteilung: **23.06.1999**
(21) Anmeldenummer: 97918049.4
(22) Anmeldetag: 21.03.1997
(51) Int. Cl.: A61K 35/78

(54) **HARPAGOSID-ANGEREICHERTER EXTRAKT AUS HARPAGOPHYTUM PROCUMBENS UND VERFAHREN ZU SEINER HERSTELLUNG**
HARPAGOSIDE-ENRICHED EXTRACT FROM HARPAGOPHYTUM PROCUMBENS AND PROCESSES FOR PRODUCING SAME
EXTRAITS D'HARPAGOPHYTUM PROCUMBENS ENRICHI EN HARPAGOSIDE ET LEURS PROCEDES DE PRODUCTION

(30) Priorität: 21.03.1996 DE 19611221; 10.12.1996 DE 19651290
(43) Veröffentlichungstag der Anmeldung: 24.02.1999
(73) Patentinhaber: Dr. Willmar Schwabe GmbH & Co., 76227 Karlsruhe (DE)
(72) Erfinder: STUMPF, Karl-Heinz, D-76228 Karlsruhe (DE); JAGGY, Hermann, D-76229 Karlsruhe (DE); OSCHMANN, Rainer, D-76829 Landau (DE); KOCH, Egon, D-76229 Karlsruhe (DE); SIMMET, Thomas, D-44780 Bochum (DE)
(74) Vertreter: Vossius, Volker, Dr.
(86) Internationale Anmeldenummer: DE9700591
(87) Internationale Veröffentlichungsnummer: WO9734565

(56) Entgegenhaltungen:
- EP-A- 0 524 873
- FR-A- 2 614 791
- JOURNAL DE PHARMACIE BELGE, Bd. 35, Nr. 2, März 1980 - April 1980, Seiten 143-149, XP002039420 MARYSE CAPRASSE: "DESCRIPTION, IDENTIFICATION ET USAGES TH RAPEUTIQUES DE LA "GRIFFE DU DIABLE": HARPAGOPHYTUM PROCUMBENS DC"
- DATABASE WPI Section Ch, Week 9309 Derwent Publications Ltd., London, GB; Class B04, AN 93-073947 XP002039421 & KR 9 205 686 B (DONGKUK PHARM CO) , 13.Juli 1992

## Beschreibung

Aufgrund langjähriger Erfahrungen werden Teezubereitungen bzw. Extrakte aus Teufelskrallenwurzeln (Radix harpagophyti) bei dyspeptischen Beschwerden sowie zur Behandlung rheumatischer Erkrankungen eingesetzt (Sticher, O., DAZ 117 (1977), 1279-1284). Die Arzneidroge besteht aus den unterirdischen Teilen, hauptsächlich den sekundären Speicherwurzeln von Harpagophytum procumbens (Volk, O.H., DAZ 104 (1964), 573-576; F.C. Czygan, Zeitschrift für Phytotherapie 8 (1987), 17-20).

M. Caprasse, J. Pharm. Belg., 1980, 35, 2, 143-149, gibt eine Übersicht über Harpagophytum procumbens und seine Eigenschaften. Die fein gepulverte Wurzel wurde mit Methanol extrahiert, der erhaltene Extrakt eingedampft, der Rückstand mit Wasser versetzt und die erhaltene wäßrige Lösung 3 mal mit einem 4:1 Gemisch aus Methylenchlorid und n-Butanol extrahiert.

R.E. Moati, FR-A-2 614 791, beschreibt eine Zusammensetzung aus 400 mg Harpagophytum procumbens, 20 mg Selen und 25 mg Zink zur Behandlung von Rheumatismus und Entzündungen.

Den Erfahrungsberichten und klinischen Beobachtungen trug die Kommission E des ehemaligen Bundesgesundheitsamtes Rechnung, indem sie 1989 eine positive Monographie "Radix harpagophyti" veröffentlichte. Danach werden Zubereitungen aus Teufelskrallenwurzeln eingesetzt bei dyspeptischen Beschwerden (Tagesdosis 1,5 g Droge) und zur unterstützenden Behandlung degenerativer Erkrankungen des Bewegungsapparates (Tagesdosis 4,5 g Droge) (Bundesanzeiger Nr. 43 vom 02.03.1989). Die Wirksamkeit konnte bisher noch nicht bestimmten Inhaltsstoffen zugeordnet werden.

Als wesentliche Inhaltsstoffe der Extrakte werden Iridoidglycoside, insbesondere Harpagosid, daneben Harpagid und Procumbid genannt. Weiterhin sind große Mengen Zucker (50-60 %), Fette, Wachse und Sterine enthalten (Steinegger, Hänsel, Lehrbuch der Pharmakognosie und Phytopharmazie, Springer Verlag Berlin, Heidelberg, New York 1988, S. 608-610). Im Handel befinden sich insbesondere Teezubereitungen (R. Jaspersen-Schib, DAZ 130 (1990), 71-73; F.-C. Czygan in M.Wichtl, Teedrogen WVA Stuttgart 1989, S. 495-497) sowie oral einzunehmende Kapsel- und Tablettenpräparate mit einfachen wäßrigen oder wäßrig alkoholischen Extrakten (Chrubasik et al., Forsch. Komplementärmed. 1996:3:57-63).

In jüngster Zeit konnte in einer klinischen Doppelblindstudie eine antirheumatische Wirksamkeit gezeigt werden. Dabei wurden Extrakte eingesetzt, die eine Dosierung von 50 mg Harpagosid pro Tag sicherstellten (S. Chrubasik, R. Ziegler in Phytopharmaka 2 - Forschung und klinische Anwendung - (Herausgeber: Loew, Rietbrock, Steinkopf Verlag Darmstadt 1996 (S. 101-114)).

Die üblichen pharmazeutischen Präparate mit einfachen Extrakten weisen Harpagosidgehalte von 1 bis 3 % auf (Chrubasik et al., Forsch. Komplementärmed. 1996:3:6-11), so daß zur Gewährleistung einer Zuführung von 50 mg Harpagosid/Tag große Extraktmengen von 1500 bis 4500 mg erforderlich werden. Dies wiederum erfordert große Arzneiformen oder häufige bzw. mehrfache Einnahme des Präparates und führt dadurch zu Verringerung der Patienten-Compliance.

Der vorliegenden Erfindung liegt die Aufgabe zugrunde, neue Extrakte mit augereichertem Harpagosidgehalt bereitzustellen, in denen Inhaltsstoffe mit fehlenden oder unerwünschten pharmakologischen Wirkungen abgereichert sind. Eine weitere Aufgabe ist es, Verfahren zur Herstellung dieser Extrakte sowie diese Extrakte enthaltende Arzneiformen bereitzustellen.

Diese Aufgaben werden gemäß Patentansprüchen 1 bis 8 gelöst.

Die Erfindung beruht auf dem überraschenden Befund, daß wäßrige oder wäßrig-alkoholische Primärextrakte, deren Harpagosidgehalt in der Regel nur 1 bis 3 % beträgt, erfindungsgemäß durch Ausrühren mit einem reinen aliphatischen Alkohol oder aliphatischen Keton oder deren Gemischen hinsichtlich Harpagosid auf Werte von mindestens 5 % stark angereichert werden können, während Inhaltsstoffe mit stimulierenden Wirkungen auf die Synthese von Thromboxan B₂ und Cysteinyl-Leukotrienen vermindert oder eliminiert werden.

Der Ausdruck "wäßrig-alkoholische Primärextrakte" bezeichnet Extrakte, die durch Extrahieren von Radix harpagophyti mit einem Gemisch aus Wasser und Ethanol erhalten werden. Die aliphatischen Alkohole enthalten 1 bis 4 C-Atome, die aliphatischen Ketone enthalten 3 bis 4 C-Atome. Bevorzugt werden zum Ausrühren Methanol, Ethanol, Propanol, Isopropanol, n-Butanol, Isobutanol, tert.-Butanol, Aceton, Butanon und deren Gemische verwendet. Besonders bevorzugt ist Ethanol. Das Ausrühren wird bei einer Temperatur im Bereich von etwa 5 bis 25°C durchgeführt. Der Ausdruck "Ausrühren" bedeutet intensives Vermischen durch Rühren.

In einer bevorzugten Ausführung wird die zerkleinerte Droge mit 20 % Ethanol/Wasser extrahiert. Anschließend wird konzentriert und der erhaltene Primärextrakt bei Raumtemperatur mit Ethanol 96 % ausgerührt. Die lösliche Fraktion wird von der unlöslichen Fraktion abgetrennt und getrocknet. Sie enthält mindestens 5 % Harpagosid.

Ein weiterer überraschender Befund war, daß die nach dem angegebenen Verfahren hergestellten Extrakte im Vergleich zu Primärextrakten, aber auch im Vergleich zu reinem Harpagosid eine höhere pharmakologische Wirksamkeit aufwiesen. Es wurde festgestellt, daß übliche Primärextrakte Inhaltsstoffe enthalten, die eine potente Stimulation der Synthese proinflammatorischer Lipidmediatoren bewirken und dadurch den erwünschten antiphlogistischen Effekten entgegenwirken. Diese Inhaltsstoffe sind in den erfindungsgemäßen Extrakten abgereichert. Die Abreicherung bzw. Entfernung dieser unerwünschten Inhaltsstoffe erfolgt durch das erfindungsgemäße Ausrühren des Primärextraktes mit dem Alkohol oder Keton oder deren Gemischen.

Die erfindungsgemäßen Extrakte können unter Zusatz üblicher pharmazeutischer Hilfsstoffe in bekannter Weise zu oral applizierbaren festen oder flüssigen Arzneizubereitungen mit antirheumatischer und antiphlogistischer Wirkung verarbeitet werden.

Die Erfindung wird anhand folgender Beispiele weiter erläutert:

### Beispiel 1

30 kg durch Mahlung zerkleinerte, getrocknete sekundäre Speicherwurzeln von Harpagophytum procumbens wurden mit 300 kg auf 85°C erhitztem vollentsalztem Wasser versetzt und zwei Stunden gerührt. Der Extrakt wurde über ein Plattenfilter unter Druck abfiltriert. Das Pflanzenmaterial wurde nochmals mit 120 kg heißem Wasser bei 85°C zwei Stunden extrahiert und der Extrakt abfiltriert. Die Extrakte aus beiden Extraktionsstufen wurden vereinigt: 348 kg. Sodann wurde der erhaltene Extrakt in einem Zentrifugalverdampfer unter Vakuum auf 19 kg mit einem Trockenrückstand von 60,5 % konzentriert. Der hierbei erhaltene Dickextrakt wurde bei max. 60°C und 20 mbar Druck getrocknet. Erhalten wurden daraus 11,4 kg Trockenextrakt mit einem Harpagosidgehalt von 2,25 % (mittels HPLC bestimmt).

### Beispiel 2

90 kg getrocknete sekundare Speicherwurzeln von Harpagophytum procumbens wurden in drei Chargen, entsprechend Beispiel 1, mit heißem Wasser extrahiert. Die vereinigten Extraktlösungen (1032 kg) wurden in einem Zentrifugalverdampfer unter Vakuum bei 180-220 mbar und 50°C auf 67 kg mit einem Trockenrückstand von 51,2 % konzentriert. Dieses Konzentrat, d.h. der Primärextrakt, wurde unter Rühren in 350 kg 95 Gew.% Ethanol bei 20°C (Raumtemperatur) langsam eingetropft. Zum Absitzen des sich hierbei bildenden Niederschlags wurde das Gemisch zwei Stunden ohne Rühren belassen. Die überstehende Lösung wurde abgehebert und in einem Zentrifugalverdampfer auf einen Trockenrückstand von 54,4 % konzentriert. In einem Vakuumtrockenschrank wurde dieses Konzentrat bei max. 60°C und 20 mbar Druck getrocknet. Es resultierten 7,56 kg harpagosidreicher Trockenextrakt mit einem Harpagosidgehalt von 7,3 % (mittels HPLC bestimmt).

### Beispiel 3

101,5 kg, über ein 7 mm-Sieb zerkleinerte, getrocknete sekundär Speicherwurzeln von Harpagophytum procumbens wurden mit 1420 kg 20 Gew.-% Ethanol zwei Stunden unter Rühren auf 75°C erwärmt. Nach Abkühlung auf 50°C wurde der Rückstand abfiltriert und nochmals mit 1010 kg 20 Gew.-% Ethanol bei 75°C extrahiert. Die vereinigten Extraktlösungen wurden in einem Zentrifugalverdampfer unter Vakuum bei 180-220 mbar/50°C auf 87 kg mit einem Trockenrückstand von 68 % konzentriert. Dieses Konzentrat (Primärextrakt) wurde unter Rühren zu 240 kg 95 Gew.-% Ethanol zugegeben. Nach dem Absitzen des Niederschlags wurde die überstehende Lösung abgetrennt und unter Vakuum bei 250 mbar in einem Zentrifugalverdampfer auf 16 kg konzentriert. Im Vakuumtrockenschrank wurde dieses Konzentrat bei max. 65°C und 20 mbar Druck getrocknet. Es resultierten 8,5 kg harpagosidreicher Extrakt mit 12,7 % Harpagosid (mittels HPLC bestimmt).

### Beispiel 4

Der gemäß Beispiel 3 bei dem Ausrühren mit Ethanol entstandene Niederschlag wurde im Vakuumschrank bei 65°C und 20 mbar Druck getrocknet. Es resultierten 21 kg einer Extraktfraktion mit 0,35 % Harpagosid.

### Beispiel 5

1,5 kg gemahlene getrocknete sekundare Speicherwurzeln von Harpagophytum procumbens wurden mit 15 kg 80 Gew.-% Ethanol eine Stunde bei 60°C extrahiert. Die Extraktlösung wurde nach dem Abkühlen über eine Filterschicht abgesaugt. Der Extraktionsrückstand wurde noch einmal mit 15 kg 80 Gew.-% Ethanol eine Stunde bei 60°C nachextrahiert. Die vereinigten Extraktlösungen wurden am Rotationsverdampfer bei einer Badtemperatur von 50 - 60°C unter Vakuum von 120 mbar konzentriert, bis kein Ethanol mehr überdestillierte.

Durch Zugabe von vollentsalztem Wasser wurde das Konzentrat auf einen Trockenrückstand von 10 % verdünnt. Diese Lösung (Primärextrakt) wurde dreimal mit jeweils 1800 g n-Butanol bei Raumtemperatur intensiv gerührt. Die gebildete Butanol-Phase (Oberphase) wurde jeweils abgetrennt. Am Rotationsverdampfer wurden die vereinigten Butanol-Phasen unter Vakuum bei 20-30 mbar bis zur Trockne eingeengt. Der Trockenextrakt wurde zerkleinert und im Vakuumtrockenschrank bei 60°C und 10 mbar nachgetrocknet. Es resultierten 61 g harpagosidreicher Extrakt mit einem Harpagosidgehalt von 19,3 % (mittels HPLC bestimmt).

### Beispiel 6

### Filmtabletten mit erfindungsgemäßem Extrakt

Filmtabletten mit 200 bzw. 400 mg des erfindungsgemäßen Trockenextraktes weisen folgende Zusammensetzung auf:

| | | |
|---|---|---|
| Trockenextrakt aus Teufelskrallenwurzel von Beispiel 5 | 200 mg | 400 mg |
| Hochdisperses Siliciumdioxid | 5 mg | 10 mg |
| Polyvinylpyrrolidon (Mol.gew. 25000) | 3 mg | 6 mg |
| Quervernetztes Polyvinylpyrrolidon | 5 mg | 10 mg |
| Magnesiumstearat | 2 mg | 4 mg |
| Cellulose, mikrokristallin | 35 mg | 70 mg |

Die Bestandteile wurden gemischt und zu Tabletten verpreßt. Die Tabletten werden mit einem Filmüberzug auf Basis Methylhydroxypropylcellulose überzogen.

### Pharmakologische Wirksamkeit verschiedener Extrakte

Die pharmakologischen Effekte auf die Bildung proinflammatorischer Lipidmediatoren wurden in humanem Vollblut nach Stimulation mit dem Calciumionophor A23187 (10 mM, 60 min bei 37°C) nach der Methode von I. Weide, K. Tschorn, T. Simmet (Thrombosis Research 67, S. 123-134 (1992)) untersucht. Eine hemmende Wirkung in diesem Modell weisen beispielsweise Glucocorticoide auf. Bestimmt wurde im Plasma die Konzentration der Cysteinyl-Leukotriene (LTC₄, LTD₄ und LTE₄) und des Thromboxans B₂ mit Hilfe eines Radioimmunoassays. Es zeigte sich, daß Harpagosid bzw. Harpagophytum-Extrakte die Biosynthese dieser Stoffe konzentrationsabhängig hemmten. Die Ergebnisse sind in Tabelle I zusammengefaßt. Wie aus der Tabelle I ersichtlich ist, war die Cysteinyl-LT-Hemmwirkung der erfindungsgemäßen Extrakte (hergestellt nach Beispiel 2 oder 3) stärker ausgeprägt als die eines Primärextraktes (hergestellt nach Beispiel 1) oder auch die von reinem Harpagosid.

Bei der Untersuchung des zur Anreicherung wirksamer Inhaltsstoffe aus dem Primärextrakt nach Beispiel 3 entfernten Niederschlages (Beispiel 4) zeigte sich, daß dieser die Biosynthese von Cysteinyl-Leuktrienen und TXB₂ deutlich stimuliert (Tabelle II) und damit den gewünschten entzündungshemmenden Effekten entgegenwirkt.

**Tabelle I**

| | | |
|---|---|---|
| Hemmung der Biosynthese von Cysteinyl-Leukotrienen und TXB₂ in humanen Vollblut nach Vorinkubation mit Harpagosid oder Harpagophytum-Extrakten (15 min bei 37°C) und anschließende Stimulation mit A23187 (10 mM, 37°C für 60 min). Angegeben ist die halb-maximale Hemmkonzentration (IC₅₀) bezogen auf den Gehalt an Harpagosid. | | |

| | IC₅₀ TXB₂ | IC₅₀ Cysteinyl-LT |
|---|---|---|
| Harpagosid | 48,6 mM | 39 mM |
| Harpagophytum-Extrakt (Primärextrakt hergestellt nach Beispiel 1) | > 100 mM | 61,7 mM |
| Erfindungsgemäßer Harpagosid-reicher Extrakt (hergestellt nach Beispiel 2) | 55,3 mM | 9,2 mM |

**Tabelle II**

| | | |
|---|---|---|
| Stimulation der Biosynthese von Cysteinyl-Leukotrienen und TXB₂ in humanen Vollblut nach Vorbehandlung mit der gemäß Beispiel 4 erhaltenen Extraktfraktion (15 min bei 37°C) und anschließender Stimulation mit A23187 (10 mM, 37°C für 60 min). Angegeben ist die prozentuale Stimulation gegenüber einen Kontrollansatz bei einer Konzentration von 1 mg/ml. | | |

| | IC₅₀ TXB₂ | IC₅₀ Cysteinyl-LT |
|---|---|---|
| Harpagophytum-Extraktfraktion von Beispiel 4 | +28,7 % | +70,5 % |

Die Analysemethoden von Thromboxan B₂ und Cysteinyl-Leukotrienen sind in folgenden Literaturstellen eingehend beschrieben:
Simmet Th., Luck W. (1989), Clotting of whole human blood induces cysteinyl-leukotriene formation. *Thromb Res* 54:423-433.
Simmet Th., Weide I. (1991), Thromboxane and cysteinyl-leukotriene formation are differentially activated in spontaneously clotting whole human blood in vitro. *Thromb Res* 62:249-261.
Weide I., Tschorn K., Simmet Th. (1992), Effects of cyclooxygenase inhibitors on ex vivo cysteinyl-leukotriene production by whole human blood allowed to clot spontaneously. Comparison to stimulated blood. *Thromb Res* 67:123-134.
Weide I., Simmet Th. (1993), Leukotriene formation by peripheral monocytes in contact-activated human blood. *Thromb Res* 71:185-192.
Weide I., Römisch J., Simmet Th. (1994), Contact activation triggers stimulation of the monocyte 5-lipoxygenase pathway via plasmin. *Blood* 83:1941-1951.

## Patentansprüche

1. Extrakt aus Harpagophytum procumbens, erhältlich durch die Schritte
a) Extrahieren der Droge mit Wasser oder einem Gemisch aus Ethanol und Wasser,
b) Konzentrieren des erhaltenen Extraktes,
c) Ausrühren des erhaltenen Konzentrats bei Temperaturen von 5 bis 25°C mit einem reinen aliphatischen Alkohol mit 1 bis 4 Kohlenstoffatomen oder einem aliphatischen Keton mit 3 bis 4 Kohlenstoffatomen oder deren Gemischen, und
d) Abtrennen der unlöslichen von der erhaltenen löslichen Fraktion, welche durch stark verminderten Gehalt von Inhaltsstoffen mit stimulierenden Wirkungen auf die Synthese von Thromboxan B₂ und Cysteinyl-Leukotrienen gekennzeichnet ist.

2. Extrakt nach Anspruch 1, erhältlich durch den zusätzlichen Schritt
e) Trocknen der erhaltenen löslichen Frakion.

3. Extrakt nach Anspruch 2 mit einem Gehalt von mindestens 5% Harpagosid.

4. Verfahren zur Herstellung eines Extraktes aus Harpagophytum procumbens nach Anspruch 1, umfassend folgende Schritte:
a) Extrahieren der Droge mit Wasser oder einem Gemisch aus Ethanol und Wasser,
b) Konzentrieren des erhaltenen Extraktes,
c) Ausrühren des erhaltenen Konzentrats bei Temperaturen von 5 bis 25°C mit einem reinen aliphatischen Alkohol mit 1 bis 4 Kohlenstoffatomen oder einem aliphatischen Keton mit 3 bis 4 Kohlenstoffatomen oder deren Gemischen, und
d) Abtrennen der unlöslichen von der erhaltenen löslichen Fraktion, welche durch einen stark verminderten Gehalt von Inhaltsstoffen mit stimulierenden Wirkungen auf die Synthese von Thromboxan B₂ und Cysteinyl-Leukotrienen gekennzeichnet ist.

5. Verfahren nach Anspruch 4, dadurch gekennzeichnet, daß zum Ausrühren Methanol, Ethanol 96%, Propanol, Isopropanol, Butanol, Butanon, Aceton oder ein Gemisch davon verwendet wird.

6. Verfahren nach Anspruch 4, umfassend den zusätzlichen Schritt
e) Trocknen der erhaltenen löslichen Fraktion.

7. Pharmazeutische Zusammensetzung, enthaltend einen Extrakt nach einem der Ansprüche 1 bis 3.

8. Verwendung des Extraktes nach einem der Ansprüche 1 bis 3 zur Herstellung einer pharmazeutischen Zusammensetzung mit antirheumatischer und antiphlogistischer Wirkung.

## Claims

1. Extract from harpagophytum procumbens, obtainable by the steps of
a) extracting the drug with water or a mixture of ethanol and water,
b) concentrating the obtained extract,
c) intensive stirring of the obtained concentrate at temperatures from 5° to 25°C with a pure aliphatic alcohol containing 1 to 4 carbon atoms or an aliphatic ketone containing 3 to 4 carbon atoms or mixtures thereof, and
d) separating the insoluble fraction from the obtained soluble fraction, which soluble fraction is characterized by a strongly reduced content of ingredients having stimulating effects on the synthesis of thromboxane B₂ and cysteinyl-leukotrienes.

2. Extract according to claim 1, obtainable by the additional step of
e) drying the obtained soluble fraction.

3. Extract according to claim 2, wherein the content of harpagoside is at least 5%.

4. Method for the preparation of an extract from harpagophytum procumbens according to claim 1, comprising the following steps:
a) extracting the drug with water or a mixture of ethanol and water,
b) concentrating the obtained extract,
c) intensive stirring of the obtained concentrate at temperatures from 5° to 25°C with a pure aliphatic alcohol containing 1 to 4 carbon atoms or an aliphatic ketone containing 3 to 4 carbon atoms or mixtures thereof, and
d) separating the insoluble fraction from the obtained soluble fraction, which soluble fraction is characterized by a strongly reduced content of ingredients having stimulating effects on the synthesis of thromboxane B₂ and cysteinyl-leukotrienes.

5. The method of claim 4, characterized in that for the intensive stirring methanol, ethanol 96%, propanol, isopropanol, butanol, butanone, acetone or a mixture thereof is used.

6. The method of claim 4, comprising the additional step of
e) drying the obtained soluble fraction.

7. Pharmaceutical composition, containing an extract according to any one of claims 1 to 3.

8. Use of the extract according to any one of claims 1 to 3 for the preparation of a pharmaceutical composition having antirheumatic and antiphlogistic effect.

## Revendications

1. Extrait d'Harpagophytum procumbens, pouvant être obtenu par les étapes de
a) extraction du principe actif avec de l'eau ou un mélange d'éthanol et d'eau,
b) concentration de l'extrait obtenu,
c) agitation intense du concentré obtenu à des températures de 5° à 25°C avec un alcool aliphatique pur contenant 1 à 4 atomes de carbone ou une cétone aliphatique contenant 3 à 4 atomes de carbone ou des mélanges de ceux-ci, et
d) séparation de la fraction insoluble de la fraction soluble obtenue, laquelle fraction soluble est caractérisée par une teneur fortement réduite en constituants ayant des effets de stimulation sur la synthèse de thromboxane B₂ et de cystéinyl-leucotriènes.

2. Extrait selon la revendication 1, pouvant être obtenu par l'étape additionnelle de
e) séchage de la fraction soluble obtenue.

3. Extrait selon la revendication 2, dans lequel la teneur en harpagoside est d'au moins 5%.

4. Procédé pour la préparation d'un extrait d'Harpagophytum procumbens selon la revendication 1, comprenant les étapes suivantes:
a) extraction du principe actif avec de l'eau ou un mélange d'éthanol et d'eau,
b) concentration de l'extrait obtenu,
c) agitation intense du concentré obtenu à des températures de 5° à 25°C avec un alcool aliphatique pur contenant 1 à 4 atomes de carbone ou une cétone aliphatique contenant 3 à 4 atomes de carbone ou des mélanges de ceux-ci, et
d) séparation de la fraction insoluble de la fraction soluble obtenue, laquelle fraction soluble est caractérisée par une teneur fortement réduite en constituants ayant des effets de stimulation sur la synthèse de thromboxane B₂ et de cystéinyl-leucotriènes.

5. Procédé selon la revendication 4, caractérisé en ce qu'on utilise pour le mélange intense du méthanol, de l'éthanol à 96%, du propanol, de l'isopropanol, du butanol, de la butanone, de l'acétone ou un mélange de ceux-ci.

6. Procédé selon la revendication 4, comprenant l'étape additionnelle de
e) séchage de la fraction soluble obtenue.

7. Composition pharmaceutique, contenant un extrait selon l'une quelconque des revendications 1 à 3.

8. Utilisation de l'extrait selon l'une quelconque des revendications 1 à 3 pour la préparation d'une composition pharmaceutique ayant un effet anti-rhumatismal et anti-inflammatoire.
